# EUROPÄISCHE PATENTSCHRIFT

(11) **EP 1 940 539 B1**
(45) Veröffentlichungstag und Bekanntmachung des Hinweises auf die Patenterteilung: **15.12.2010**
(21) Anmeldenummer: 06790927.5
(22) Anmeldetag: 02.10.2006
(51) Int. Cl.: B01F 11/00, B01F 11/04, B01F 3/04

(54) **EINWEGGEBINDE MIT RÜHRER**
ONE-TRIP CONTAINER COMPRISING A STIRRING DEVICE
CONTENANT JETABLE A AGITATEUR

(30) Priorität: 03.10.2005 CH 15882005; 20.04.2006 CH 6492006
(43) Veröffentlichungstag der Anmeldung: 09.07.2008
(73) Patentinhaber: Zeta Biopharma GmbH, 8144 Tobelbad (AT)
(72) Erfinder: MEIER, Hans, Peter, CH-8855 Wangen/Nuolen (CH)
(74) Vertreter: Becker, Thomas
(86) Internationale Anmeldenummer: PCT/CH2006/000536
(87) Internationale Veröffentlichungsnummer: WO 2007/038893

(56) Entgegenhaltungen:
- EP-A1- 0 317 811
- CH-A- 283 308
- CH-A- 283 308
- DE-B- 1 027 640
- DE-B- 1 027 640
- DE-U- 7 504 145
- FR-A- 2 289 094
- US-A- 2 499 203
- US-A- 2 615 692
- US-A- 3 560 162
- US-A1- 2003 198 406
- US-A1- 2003 198 406

## Beschreibung

Die Erfindung betrifft ein Einweg-Gebinde aus Kunststoffmaterial, wie ein Einweg-Mischgebinde mit einem Mischer für Fluide, insbesondere zum Mischen von sterilen Flüssigkeiten für die chemische, biotechnologische, pharmazeutische und Lebensmittelindustrie.

Bei der sterilen Produktion, insbesondere in der pharmazeutischen und biotechnologischen Industrie werden an allen Oberflächen, die mit dem Produkt oder Vorstufen davon in Kontakt kommen, höchste Ansprüche gestellt, da über die Oberflächen keine Verschmutzung in das Produkt gelangen darf. Das Interesse nach sterilen Einwegbehältern ist in den letzten Jahren gestiegen.

Die US 2003/0198406 A1 zeigt einem flexiblen Behälter mit einem darin uniaxial bewegbaren Rührwerk gemäß den Oberbegriff des Anspruchs 1. Die Tragachse des Rührorgans wird über Manschetten oder ähnliche Bauteile mit einem Diaphragma verbunden, welches an der Behälterwand angeflanscht ist.

Es ist Aufgabe der Erfindung, ein Gebinde zur Verfügung zu stellen, das für den Sterilbereich akzeptabel ist und eine Misch- oder Rührleistung aufweist, die höchsten Ansprüchen genügt.

Dies wird durch ein Einweggebinde gemäß Patentanspruch 1 gelöst.

Die Vorrichtung zum Rühren, Mischen, Emulgieren etc. (im Folgenden werden zusammenfassend nur noch die Begriffe Rührer und Rühren gebraucht) von Flüssigkeiten umfasst mindestens ein Rührorgan, das auf einer Tragachse angebracht ist. Die Tragachse durchsetzt die Gebindewand, so dass das Rührorgan im Inneren des Gebindes ist, während ein entgegengesetzter Bereich der Tragachse über einen externen Antrieb angetrieben werden kann. Das Rührorgan ist so gewählt, dass das zu rührende Fluid nur durch reziproke Auf- und Abbewegungen des Rührorgans gerührt werden kann und keine Drehbewegungen zum Rühren nötig sind. Die Flexibilität der Gebindewand und die rein axial gerichtete Bewegung der Tragachse erlaubt es, Tragachse und Gebindewand integral zu fertigen und es kann auf jegliche zusätzliche Form der Abdichtung verzichtet werden. Das Gebinde ist weiterhin hermetisch gegenüber der umgebenden Atmosphäre abgeschlossen.

Ein weiterer Vorteil liegt in den günstigen Produktionskosten.

Bevorzugte Ausführungsformen des erfindungsgemäßen Rührers werden nachstehend anhand der Zeichnungen beschrieben. Es zeigen:
- Figur 1:: ein Gebinde mit einem Rührorgan gemäß einer ersten Ausfüh- rungsform in einem Stützbehälter, teilweise geschnitten darge- stellt,
- Figur 2:: ein Gebinde mit einem Rührorgan mit zwei Rührerplatten,
- Figur 3:: ein Gebinde mit einem Rührorgan mit innenliegendem Kanal,
- Figur 4:: ein Gebinde mit einem Rührorgan zum radialen Rühren,
- Figuren 5a, 5b:: eine Ausführungsformen eines Rührorgan mit den damit erzeugbaren Strömungen in stark vereinfachter Form,
- Figur 6a:: ein Rührorgan mit einer Detailvergrößerung des Verbindungs- bereiches zwischen Gebindewand und Rührorgan, wobei diese auf der linken Seite weggelassen ist,
- Figur 6b:: ein Rührorgan im Bereich der Verbindung zwischen Gebindewand und Flansch des Rührorgans,
- Figur 6c:: ein Rührorgan im Bereich einer direkten Befestigung der Gebindewand an der Tragachse des Rührorgans,
- Figur 6d:: ein Rührorgan im Befestigungsbereich der Gebindewand an der Tragachse.

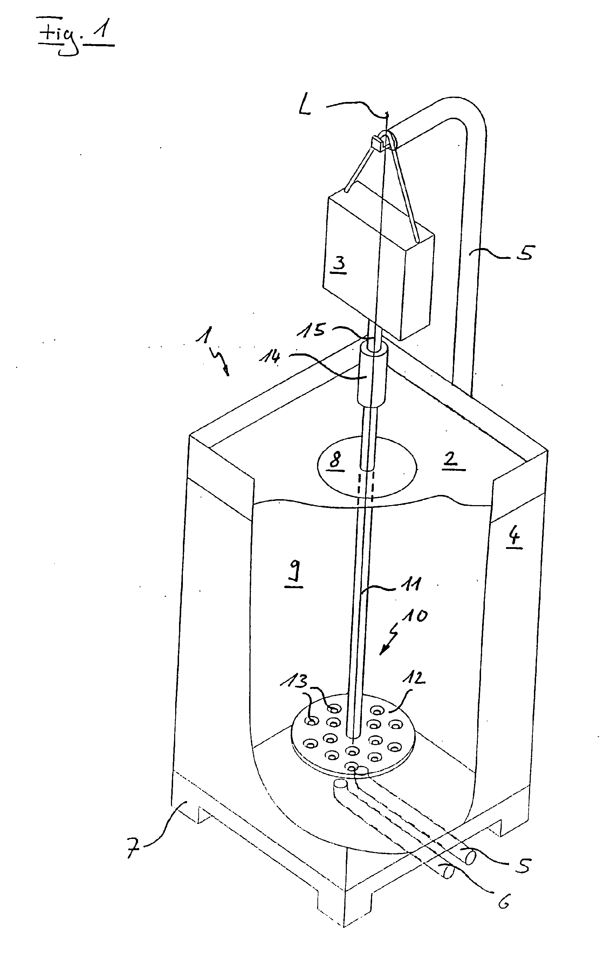

In Figur 1 ist ein flexibles Einweggebinde 1 skizziert, das von einem stützenden Behälter 4, zum Beispiel einem rechteckigen Metallbehälter, aufgenommen und stabilisiert ist. Der Behälter 4 weist in der dargestellten Ausführungsform eine Bodenplatte 7 auf, die mit Durchbrüchen für Zu- und/oder Ableitungen 5, 6 des Gebindes versehen ist. Der Behälter kann einen Doppelmantel mit einer Temperiereinrichtung umfassen. Die Form ist angepasst an das Einweggebinde von kreiszylindrisch bis vieleckig wählbar. Das Einweggebinde ist aus transparentem Folienmaterial gefertigt und kann an der Unterseite mit Anschlüssen 5, 6 zum Zu- und/oder Ableiten von Material versehen sein. Das Einweggebinde 1 ist in der gezeigten Ausführungsform mit einem Rührorgan 10 versehen.

Eine Rührerachse 1 1, auch Tragachse genannt, durchsetzt im oberen Bereich die Gebindewand 2. An einem unteren Ende der Tragachse 11 ist eine Rührerplatte 12 angeformt, die im Wesentlichen senkrecht zur Tragachse 11 steht. Am entgegengesetzten oberen Ende ist die Tragachse 11 über eine Kupplung mit einer Antriebsachse 15 eines Antriebs 3 verbunden, der über einen Arm 5 am Behälter 4 aufgehängt ist. Durch den Antrieb 3 lässt sich die Tragachse 11 in eine Auf- und Abbewegung entlang ihrer Längsachse L versetzen. Die Tragachse 11 überträgt diese Auf- und Abbewegung oder Vibration auf die Rührerplatte 12, die an ihrem entgegengesetzten Ende angebracht ist. Für viele Anwendungen genügt ein ungeregelter Antrieb, der in Abhängigkeit von der Netzfrequenz mit einer Frequenz von annähernd 50 Hz arbeitet. Der Hub lässt sich mit einfachen mechanischen Mitteln einstellen. Bei großtechnischen Anwendungen haben sich Linearmotoren bewährt, deren Frequenz und Hub in einem weiten Bereich variiert werden können.

Die Rührerplatte 12 ist mit einer Anzahl von Durchströmkanälen 13 versehen, die jeweils konische Mantelflächen 16 aufweisen.

Die Flüssigkeit wird durch die reziproke Auf- und Abbewegung des Rührorgans in gewünschter Weise in Bewegung versetzt, ohne dass eine Drehbewegung des Rührorgans zum Rühren nötig ist. Für die dargestellte Ausführungsform liegt die Amplitude der Vibration, respektive die Auslenkung der Rührerplatte 12, im Bereich etwa 1.5 mm.

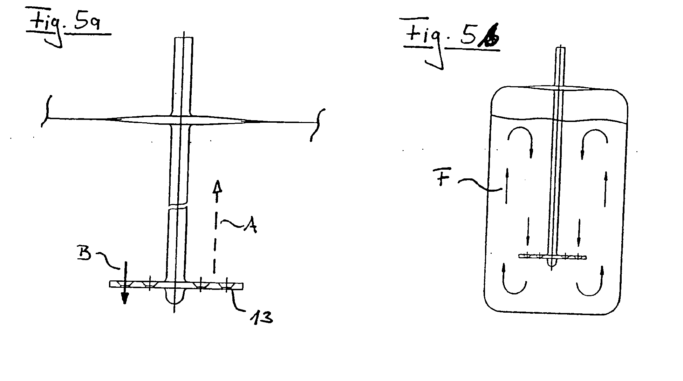

Mit dem strichlinierten Pfeil A ist die Aufwärtsbewegung der Rührerplatte 12 angedeutet, und mit Pfeil B die Pumprichtung der Flüssigkeit. In Figur 5b ist durch Pfeile angedeutet, welche Strömung sich in einer Flüssigkeit F im Gebinde 1 durch das Auf- und Abbewegen des Rührorgans gemäß Figur 5a erzeugen lässt.

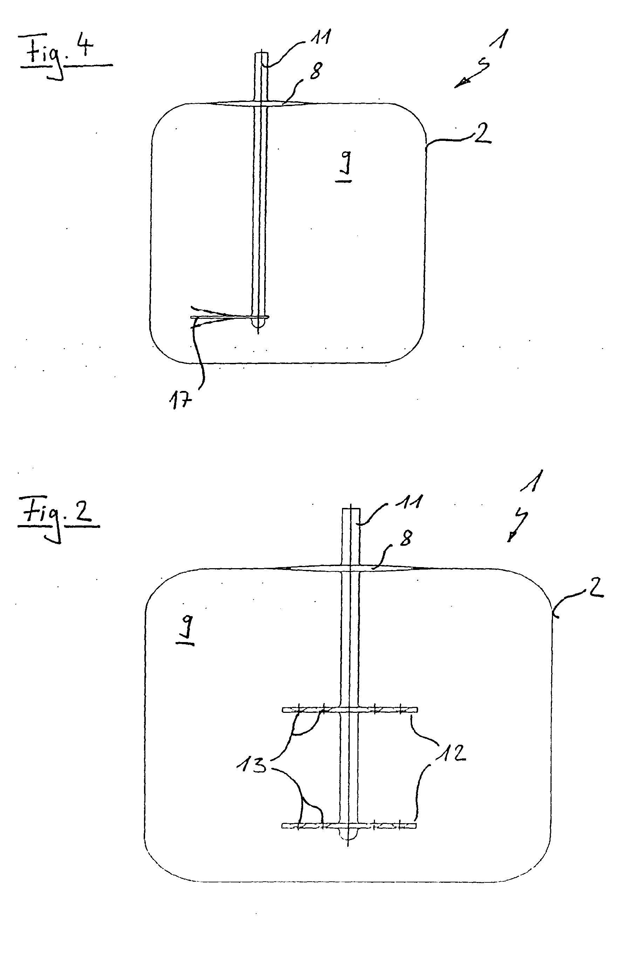

In den Figuren 2 bis 5 ist eine Anzahl von bevorzugten Ausführungsformen für Rührorgane und Rührerplatten dargestellt. Für Gebindevolumen von 0,1 bis 50 Liter haben sich Plattendurchmesser von 23, 45, 55, 65 oder 85 mm als vorteilhaft erwiesen. Für Gebindevolumen von 20 bis 150 L Plattendurchmesser: 100 oder 135, für Volumen von 50 bis 800 L Plattendurchmesser: 150 und 200 mm, für Volumen von 500 bis 2.500 L Plattendurchmesser: 300 und 380 mm. Die Plattenabmessungen werden je nach Rühraufgabe und Viskosität entsprechend ausgelegt. Am Außendurchmesser der Rührerplatte kann ein konischer Rand oder Trichter von 5 bis 20 mm Höhe und einer Neigung von 30 bis 45 Grad integriert sein, der den Rühreffekt zusätzlich verstärkt.

Wie aus Figur 2 ersichtlich, lassen sich auf der Tragachse auch mehrere Rührerplatten anbringen.

In einer weiteren Ausführungsform steht die Rührerplatte nicht senkrecht auf der Tragachse, sondern ist parallel dazu angeordnet.

In Figur 4 ist die Rührerplatte als elastisch deformierbare Platte ausgebildet. Sie wird durch die Vibrationsbewegung A" der Tragachse ihrerseits in Schwingung gebracht und/oder in eine Eigenschwingung versetzt. Bei einer Ausführungsform mit einer linksseitig angeordneten Platte 17, wie in Figur 4 gezeigt ist, wird die Flüssigkeit bei Vibration A" der Tragachse hauptsächlich radial in Richtung B" weggedrückt. Die Auslenkung der elastischen Platte 17 nach oben und unten ist in Figur 4 strichliniert angedeutet.

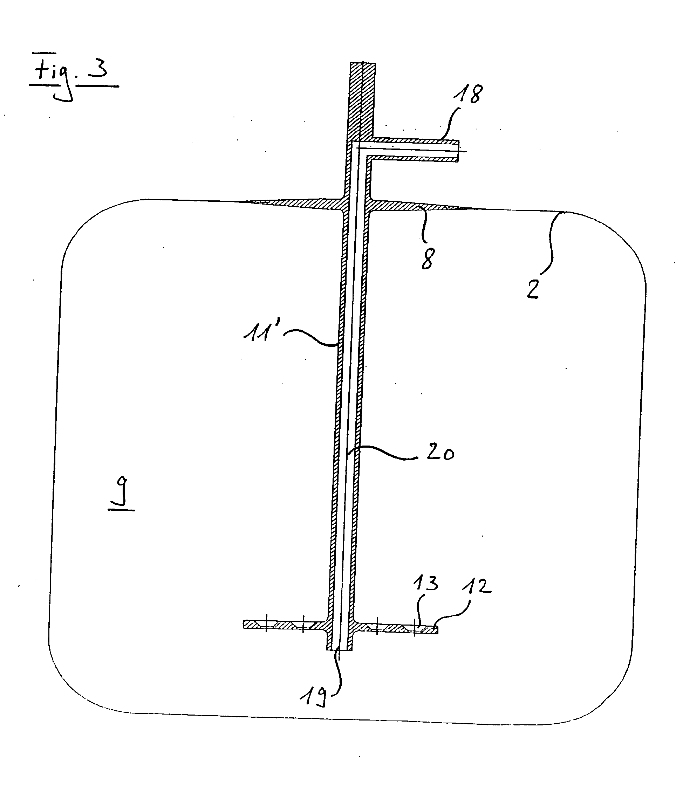

Figur 3 zeigt, dass die Tragachse 11' als Rohr mit einem zentralen Hohlraum 20 ausgestaltet ist. Im oberen Bereich der Tragachse 11', außerhalb des Gebindes, ist seitlich ein Anschlussstutzen 18 angeordnet, über den Gase, Flüssigkeiten oder fließfähige Substanzen in das Innere des Gebindes zugeführt oder aus diesem abgesaugt werden können. Das Kanalende 19 ist am unteren Ende des Tragrohrs 11' angeordnet. Da es die flexible Gebindewand zulässt, das Tragrohr bis zum Boden des Gebindes herabzubringen und dort sogar hin- und herzubewegen, kann beim Entleeren des Gebindes Flüssigkeit nahezu vollständig aus dem Inneren des Gebindes abgesaugt werden. In einem Gebinde gemäß dieser Ausführungsform kann je nach Anwendungsbereich im einfachsten Fall auf alle weiteren Anschlüsse und/oder Zugänge zum Inneren des Gebindes verzichtet werden. Dies ist wiederum nicht nur aus fertigungstechnischen- und Kostengründen interessant, sondern vor allem für Sterilanwendungen vorteilhaft.

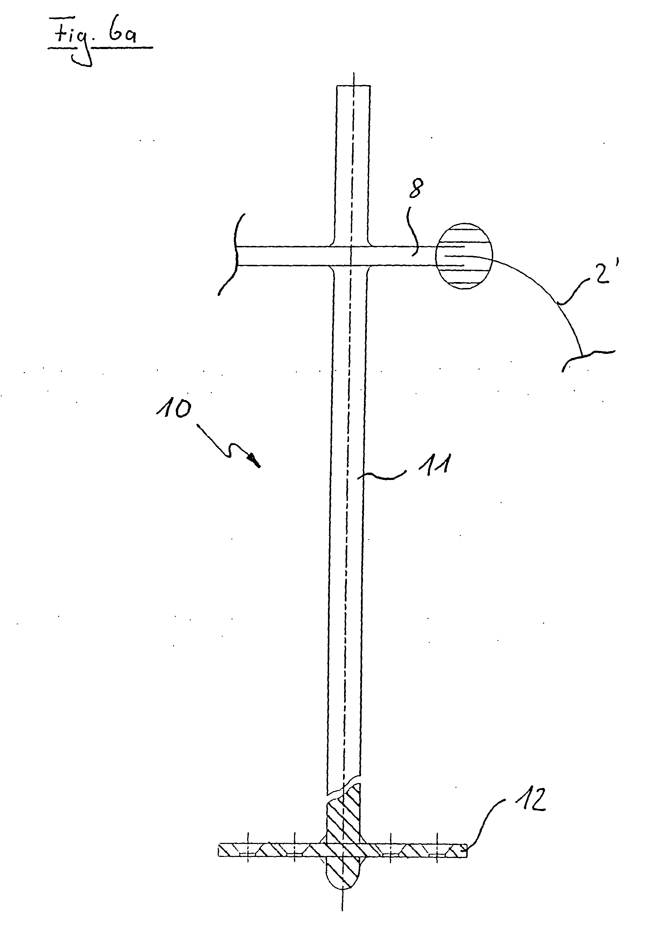

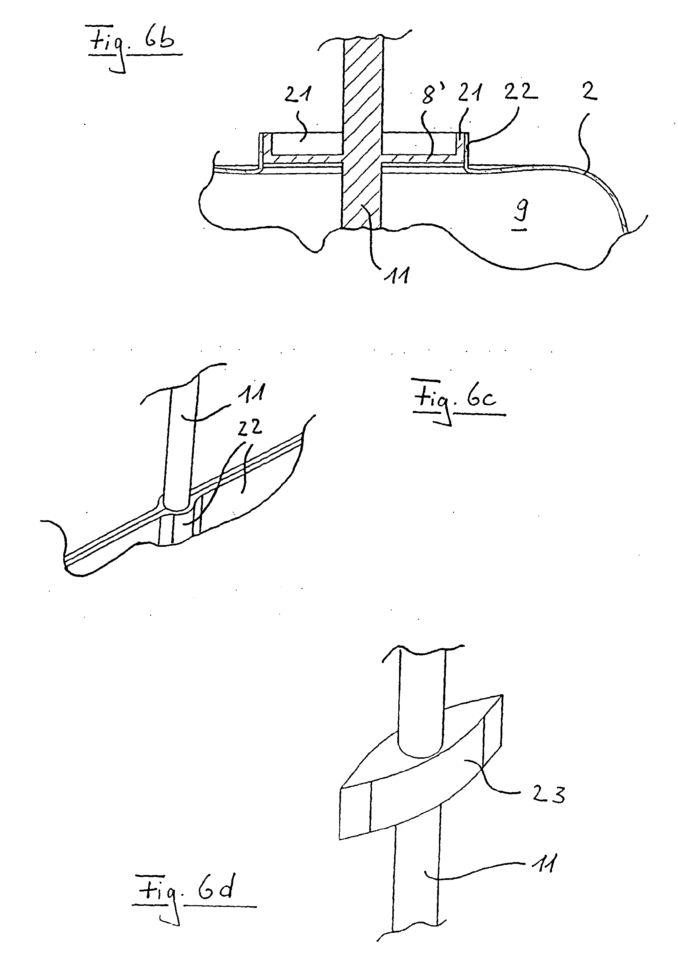

In Figur 6 sind verschiedene Möglichkeiten dargestellt, wie das eigentliche Rührorgan mit der Gebindewand dichtend verbunden wird. In Figur 6a steht ein Flansch 8 senkrecht auf der Tragachse 11. Im peripheren Bereich des Flansches 8 wird die Gebindewand vorzugsweise durch Schweißen oder Kleben dicht mit dem Flansch verbunden, kann aber auch durch geeignete Mittel mit diesem verklemmt oder verflanscht werden. In der Detailvergrößerung ist angedeutet, dass die Gebindewand 2' fünflagig aufgebaut ist, wobei die beiden äußeren Schichten (zum Beispiel eine PET- und eine PA-Lage) an der Oberseite des Flansches befestigt werden, die mittlere Schicht (zum Beispiel ein "Tie-Layer") im Flansch eingeschweißt oder -geklebt und die inneren Schichten (zum Beispiel eine EVOH- und eine ULPDE-Lage) an der Unterseite des Flansches befestigt werden. Bei einstückig vorgefertigten Gebinden hat es sich als vorteilhaft erwiesen, den Flansch mit einer peripheren umlaufenden, nach oben stehenden Rippe 21 zu versehen, an der sich ein Kragen 22 der Gebindewand 2 sehr gut befestigen lässt, da der Verbindungsbereich beidseitig gut zugänglich ist. Das Rührorgan ist vorzugsweise aus PE, PP, PEEK oder PVDF hergestellt. Rührorgane für kleinvolumige Anwendungen sind vorzugsweise einstückig gespritzt, während größere Rührorgane mehrstückig gefertigt sein können. So lässt sich um Beispiel die Tragachse strangextrudieren und die separat gefertigten, vorzugsweise im Spritzguss hergestellten Rührerplatten, Flansche, Anschlussstutzen und/oder Einsätze werden in einem zweiten Schritt auf der Tragachse angebracht.

In den Figuren 6c, 6d sind weitere Ausführungsformen gezeigt, bei denen die Gebindewand im Öffnungsbereich direkt mit der Tragachse verschweißt/verklebt ist. Bei der Ausführungsform gemäß Figur 6d ist an der Tragachse ein doppelaxtförmiger Einsatz 23 angeformt, der das Befestigen der Gebindewand an der Tragachse erleichtert. Zum Einsetzen des Rührorgans genügt in beiden Fällen jeweils ein Schnitt oder eine vorgefertigte Öffnung mit ausreichender Größe in der oberen Gebindewand. Nach Einsetzen des Rührorgans wird die Gebindewand an der Tragachse befestigt und gleichzeitig oder anschließend werden die noch freien Schnittkanten der Gebindewand zum Verschließen des Schnittes miteinander verbunden. Vorzugsweise werden die Verbindungen mittels Verschweißen erstellt.

Soll über den Hohlraum 20 begast werden, so sind vorzugsweise eine Vielzahl von entsprechenden Öffnungen im Rohr 11' angeordnet, durch die der Flüssigkeit Gas zugeführt werden kann. Die kommunizierende Verbindung, die über den Kanal 20 zwischen Stutzen 18 und Kanalende 19 respektive dem Gebindeinnenraum 9 besteht, kann durch geeignete Mittel im Kanal oder an dessen Enden dicht verschließbar ausgestaltet sein.

## Patentansprüche

1. Einweg-Gebinde (1) aus Kunststoffmaterial für sterile Flüssigkeiten für die chemische, biotechnologische, pharmazeutische und Lebensmittel-Industrie mit einem Rührorgan (10), dessen Tragachse (11, 11') eine flexible Gebindewand (2) durchsetzt, wobei ein im Inneren (9) des Gebindes (1) angeordneter Bereich der Tragachse (11) mindestens eine Rührerplatte (12, 12', 17) trägt und ein Bereich der Tragachse (11) außerhalb des Gebindes (1) mit einem Antrieb (3) für eine nur reziproke ein axial gerichtete Auf- und Abbewegung des Rührorgans (10) verbindbar ist, wobei die Tragachse (11, 11') integral mit der Gebindewand (2) verbunden ist, **gekennzeichnet durch** folgende Merkmale:
<a>) die Tragachse (11') ist als Rohr mit einem zentralen Hohlraum (20) ausgestaltet,
<b>) im oberen Bereich der Tragachse (11), außerhalb des Gebindes, ist seitlich ein Anschlussstutzen (18) angeordnet, über den Gase, Flüssigkeiten oder fließfähige Substanzen in das Innere des Gebindes zugeführt oder aus diesem abgesaugt werden können,
<c>) ein Kanalende (19) des zentralen Hohlraums (20) ist am unteren Ende der Tragachse (11') angeordnet.

2. Gebinde (1) nod Anspruch 1, mit folgenden Merkmal: die Gebindewand (2) ist im Durchsetzungsbereich mit der Tragachse (11, 11') oder einem Flansch der Tragachse verschweißt, verklebt, verklemmt oder verflanscht.

3. Gebinde (1) nach Anspruch 1, dessen Tragachse (11, 11') aus Kunststoff, vorzugsweise aus PE, PP, PEEK oder PVDF gefertigt ist.

4. Gebinde (1) nach Anspruch 1, dessen von der Tragachse durchsetzte Gebindewand (12) einen mehrschichtigen Aufbau aufweist.

5. Gebinde (1) nach Anspruch 4, dessen mehrschichtiger Aufbau eine PET-Schicht, eine PA-Schicht, eine EVOH-Schicht und eine ULPDE-Schicht umfasst.

6. Gebinde (1) nach Anspruch 1, dessen Tragachse (11, 11') und damit das mindestens eine daran angeordnete Rührorgan (12) vom Antrieb (3) in eine axial gerichtete Auf- und Abbewegung mit einer Frequenz im Bereich von minimal 10 Hz bis maximal 500 Hz versetzbar ist.

## Claims

1. A one-way container (1), made of plastic material, for sterile liquids for the chemical, biotechnological, pharmaceutical and food industry, comprising a stirring unit (10) the supporting shaft (11, 11') of which penetrates a flexible container wall (2), wherein an area of the supporting shaft (11) arranged inside (9) the container (1) supports at least one stirring plate (12, 12', 17) and an area of the supporting shaft (11) located outside the container, can be connected to a drive (3) for a mere reciprocal up and down movement of the stirring unit (10) in an axial direction wherein the support shaft (11, 11') is integrally connected to the container wall (2), **characterized by** the following features:
a) the supporting shaft (11') is realized as a pipe with a central cavity (20),
b) a connector (18) is arranged laterally in the upper region of the supporting shaft (11), outside the container, through which gases, liquids or flowable substances can be supplied to the inside of the container or removed therefrom by suction,
c) a channel end (19) of the central cavity (20) is arranged at the lower end of the support shaft (11').

2. A container (1) according to claim 1 with the following feature: the container wall (2) is welded, glued, clamped or flanged in the penetration area to the supporting shaft (11, 11') or to a flange of the supporting shaft.

3. A container (1) according to claim 1 wherein the supporting shaft (11, 11') is made of plastic material, preferably of PE, PP, PEEK or PVDF.

4. A container (1) according to claim 1, wherein the container wall (12) penetrated by the supporting shaft has a multi-layer construction.

5. A container (1) according to claim 4, wherein the multi-layer construction has a PET-layer, a PA-layer, a EVOH-layer and a ULPDE-layer.

6. A container (1) according to claim 1, wherein the supporting shaft (11, 11') and insofar the stirring unit (12) arranged thereon can be moved up-and-down by a drive (3) in an axial direction with a frequency in the range of minimum 10 Hz to maximum 500 Hz.

## Revendications

1. Contenant jetable (1) en matière plastique pour liquides stériles pour l'industrie chimique, biotechnologique, pharmaceutique et agro-alimentaire avec un élément agitateur (10), dont l'axe porteur (11, 11') traverse une paroi souple du contenant (2), une zone de l'axe porteur (11) située à l'intérieur (9) du contenant (1) portant au moins une plaque d'agitateur (12, 12', 17) et une zone de l'axe porteur (11) extérieure au contenant (1) étant susceptible d'être reliée à un entraînement (3), pour un déplacement ascendant et descendant uniquement réciproque, en direction axiale de l'élément agitateur (10), l'axe porteur (11, 11') étant intégralement relié à la paroi du contenant (2), **caractérisé par** les attributs suivants
(a) l'axe porteur (11') est conçu sous la forme d'un tube avec une cavité (20) centrale,
(b) dans la zone supérieure de l'axe porteur (11), à l'extérieur du contenant, est disposée latéralement une tubulure de raccordement (18) par l'intermédiaire de laquelle des gaz, des liquides ou des substances fluides peuvent être amenés à l'intérieur du contenant ou aspirés à partir de celui-ci,
(c) une extrémité du canal (19) de la cavité centrale (20) est disposée sur l'extrémité inférieure de l'axe porteur (11').

2. Contenant (1) selon la revendication 1, avec l'attribut suivant :
dans la zone de traversée, la paroi du contenant (2) est soudée, collée, serrée ou bridée sur l'axe porteur (11, 11') ou sur une bride de l'axe porteur.

3. Contenant (1) selon la revendication 1, dont l'axe porteur (11, 11') est fabriqué en matière plastique, de préférence en PE, en PP, en PEEK ou en PVDF.

4. Contenant (1) selon la revendication 1, dont la paroi de contenant (12) traversée par l'axe porteur présente une structure multicouche.

5. Contenant (1) selon la revendication 4, dont la structure multicouche comprend une couche de PET, une couche de PA, une couche d'EVOH et une couche d'ULPDE.

6. Contenant (1) selon la revendication 1, dont l'axe porteur (11, 11') et donc l'au moins un élément agitateur (12) disposé sur ce dernier est susceptible d'être amené par l'entraînement (3) dans un déplacement ascendant et descendant en direction axiale, à une fréquence de l'ordre d'un minimum de 10 Hz à un maximum de 500 Hz.
